# EUROPEAN PATENT SPECIFICATION

(11) **EP 0 920 853 B1**
(45) Date of publication and mention of the grant of the patent: **21.01.2004**
(21) Application number: 98203887.9
(22) Date of filing: 19.11.1998
(51) Int. Cl.: A61K 7/13

(54) **Hair dye compositions**
Haarfarbemittel
Compositions pour la teinture des cheveux

(30) Priority: 19.11.1997 US 972979
(43) Date of publication of application: 09.06.1999
(73) Proprietor: P&G-Clairol, Inc., Stamford, CT 06922 (US)
(72) Inventor: Wong, Michael Y. M., Easton, Connecticut 06612 (US); Pohl, Stanley, Scarsdale, New York 10583 (US)
(74) Representative: Adams, Harvey Vaughan John

(56) References cited:
- US-A- 5 344 463
- US-A- 5 529 583
- US-A- 5 670 698
- US-A- 5 672 180

## Description

### FIELD OF INVENTION

This invention relates to oxidative aqueous, hair coloring compositions which produce a vivid, intense dyeout having superior wash fastness and high resistence to light fading.

### BACKGROUND OF INVENTION

Modern hair dyeing methodology has developed from its initiation in the 1950's to the point where, following shampoos and conditioners, today it is the third largest product type in the hair care category.

The most commonly used method of dyeing hair, particularly human hair, is oxidative dyeing in which a mixture of aromatic compounds, generally of the benzenoid series, containing a plurality of nuclear amino and hydroxy functions, and which are essentially colorless, are converted by coupling reactions and oxidative processes, both of which are well known to those skilled in the art, to a blend of colored compounds within the hair fibers. Shortly before use, the colorless aromatic compounds, in a suitable base formulation, are normally mixed with hydrogen peroxide or other strong oxidizing agents. The colored compounds or dyes are typically formed by oxidative coupling between primary intermediates (usually diaminobenzenes or aminophenols) and couplers which are phenols or related cyclic compounds. Various shades are developed by using mixtures containing more than one of both the intermediate and the coupler.

Because of their low molecular weights and water solubility the primary intermediates and couplers diffuse easily into the hair where the coupling reaction takes place. The colored products developed by oxidation, however, remain trapped in the hair because of their higher molecular weights, relative insolubility in water and absorptive affinity to the internal hair surface. This is the basis for permanent tints and toners which, ideally, last for the life of the hair and are relatively unaffected by light, shampooing and perspiration.

The practice of oxidative hair coloring is well known and need not be described in detail. Typically, it involves the use of a two part system. One part, the dye component, contains at least one coupler/primary intermediate combination which, before use is mixed with a second part, which is a developer formulation containing an oxidizing agent, to form the dye composition. Large numbers of useful combinations are known and described for example, in the following representative United States Patents:
5,032,138; 5,393,305;
5,344,463; 4,566,876;
5,393,305; 4,883,656;
US 5,672,180 discloses 2-methyl-1-naphthol couplers in particular.

Although hydrogen peroxide is the most widely employed oxidant, other oxidizing agents are known and useful in this invention. These include alkali and alkaline earth metal chlorites which are described in U.S. Patent 5,032,138. Other oxidizing agents employed in oxidative hair coloring include; urea peroxide, melamine peroxide and perborates and percarbonates such as sodium perborate and sodium percarbonate. All such oxidants are useful in the practice of this invention and will be employed in the usual manner. For example, if a peroxide is used, the concentration in the developer may be from about 0.5% to about 20% by weight, preferably 0.5% to about 15% by weight. If the preferred hydrogen peroxide is employed, the concentration will be from about 0.5% to about 12% by weight, preferably 3% to 9% by weight.

Unless otherwise indicated, all percents in disclosure and claims are percent by weight based on the total weight

To be useful for coloring hair, oxidative dye formulations should meet at least the following conditions:
1. They must provide a range of color shades that are acceptable to practitioners of hair coloring.
2. The couplers and primary intermediates must be properly dispersed to ensure that they rapidly penetrate into the hair.
3. The dye component should have the necessary properties so that when it is mixed with the developer, the resulting mixture has the desired rheological properties. The mixture preferably is thin enough to be able to spread onto the hair, but thick enough to stay in place during the color development period. If thickened, the mixture should also be readily rinsable from the hair with water.
4. The formulations should not be irritating to the scalp.

### THE FIGURE

The figure is a graph showing the variation of red intensity with the HLB Index. Red intensity "a" is determined by conventional methods with a Minolta CM2002 Colorimeter.

### DETAILED DESCRIPTION OF THE INVENTION

The present inventors have surprisingly discovered that certain heretofore not specifically disclosed dye intermediates of a class, previously known to yield useful colors are particularly useful when delivered from a vehicle having certain required characteristics. When so delivered, vivid intense colors are produced. Moreover, the dyeouts exhibit superior wash fastness and high resistance to light fading.

The characteristics of the vehicle that are essential to securing the benefits of the invention include the amounts of water, organic solvents, and also the nature of the surfactants in the composition. Specifically, the hair dyeing efficiency of the vehicle is determined by the "Cumulative HLB Index" of the final composition.

"Cumulative HLB Index" is defined as the sum of the HLB indexes of each ingredient in the formulation.

Ingredients present in low concentration can be ignored for purposes of this calculation as they will mathematically have little or no impact on the Cumulative HLB index.

"HLB Index" of an ingredient is determined by dividing its formula weight percent by its HLB. Thus, the Cumulative HLB Index would be equal to Wt% of ingredient 1/HLB value of ingredient 1+ Wt% of ingredient 2/HLB value of ingredient 2+........ etc.

The following is an example of how the Cumulative HLB Index is calculated for a composition containing water, Surfactant A and Surfactant B.

| Ingredients | Wt% | HLB value of the ingredient |
|---|---|---|
| Water | 80 | 47 |
| Surfactant A | 15 | 20 |
| Surfactant B | 5 | 10 |

Thus, "Cumulative HLB Index" = 80/47 + 15/20 + 5/10 = 1.7 + 0.75 + 0.5 = 2.95

Other components in typical compositions contribute to the Cumulative HLB Index. As stated earlier, when they are present in low concentration and have low HLB value, their contribution to the Cumulative HLB Index of the formulation would be negligible and consequently they may be ignored for purposes of calculating the formulations Cumulative HLB Index.

HLB is a concept well known and understood by the skilled artisan. It is an expression of the relative simultaneous attraction of material, particularly a surfactant for water and oil. A surfactant having a high HLB is considered hydrophilic. A surfactant having a low HLB is considered lipophilic.

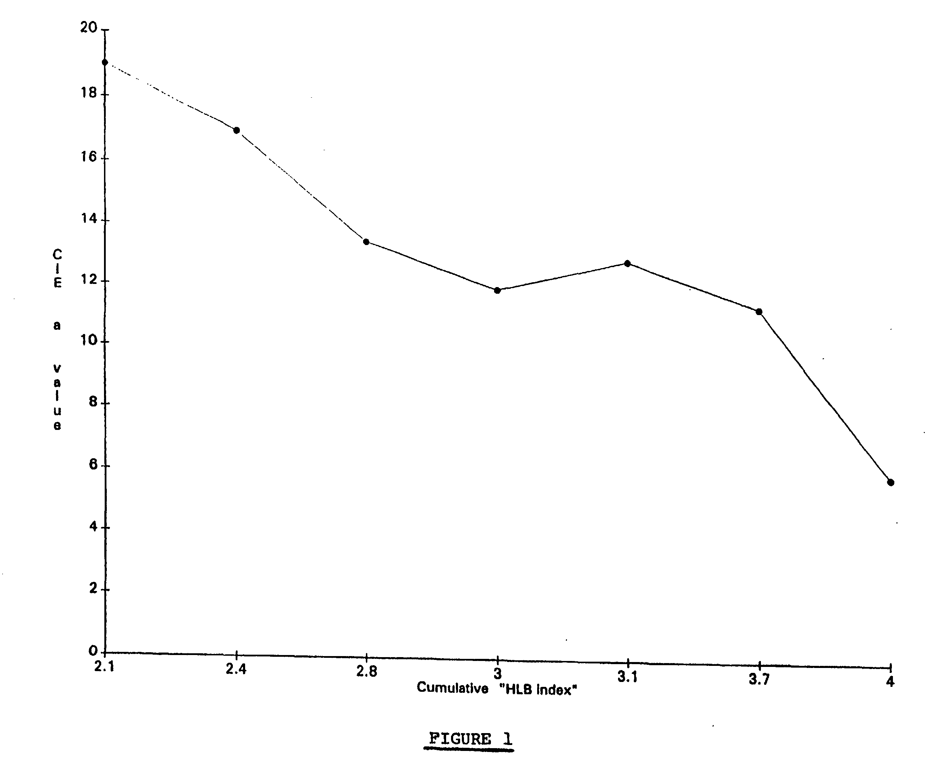

With the novel compositions of this invention, the Cumulative HLB Index will be up to about 2.5, although useful results can be obtained with somewhat higher values. As is seen in the accompanying Figure 1, wherein CIE 'a' value (in other words red intensity) is plotted against Cumulative HLB Index, the red intensity rapidly decreases as the Cumulative HLB Index approaches 2.8. The preferred range of the Cumulative HLB Index is from about 2.1 to about 2.5.

The novel oxidative dye compositions of this invention are formed by mixing at least one 2-substituted-1-naphthol coupler of formula I and at least one primary intermediate of formula II with a conventional developer, either on the hair or just prior to contacting the hair.

The 2-substituted 1-naphthols useful in the invention conform to the formula I: 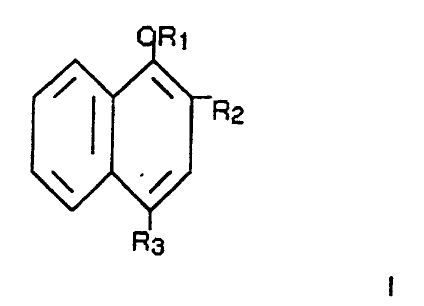 in which R₁ denotes hydrogen or a C₁-C₆ acyl group, R₂ denotes a C₁-C₆ alkyl group or a C₁-C₆ alkyl group substituted with at least one hydroxyl group, and R₃ denotes hydrogen, a C₁-C₆ alkyl group or a C₁-C₆ alkyl group substituted with at least one hydroxyl group.

The primary intermediates useful in the invention conform to the formula II: 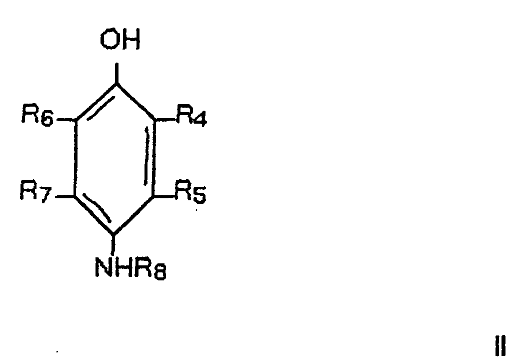 in which R₄, R₅, R₆ and R₇, independently of one another, denote hydrogen, a C₁-C₆ alkyl group or a C₁-C₆ alkyl group substituted with at least one hydroxyl group, and R₈ denotes hydrogen or C₁-C₆ alkyl group.

Presently preferred compounds within the scope of formula I include 2-methyl-1-naphthol, 2-ethyl-1-naphthol, 2-propyl-1-naphthol, 2-. hydroxymethyl-1-naphthol, 2-hydroxyethyl-1-naphthol, 1-acetoxy-2-methyl-naphthalene and 4-methoxy-2-methyl-1-naphthol. Most preferred is 2-methyl-1-naphthol or its acetoxy derivative.

Amongst the preferred compounds of formula II are 4-aminophenol, 2-methyl-4-aminophenol, 2-methyl-4-(N-methyl) aminophenol, 2-methyl-4-(N-ethyl) aminophenol and 2-methyl-4-amino-5-hydroxyethyl-phenol. The most preferred compounds are 4-aminophenol and 3-methyl-4-aminophenol.

As will be seen, compounds of formulas I are 2-substituted-1-naphthols. Compounds of formula II are 4-aminophenols which may be substituted at one or more positions. These compounds of formulas 1 and 2 are the essential dye precursors. When these specified dye precursors are coupled under oxidizing conditions and in a final composition having the characteristics called for by this invention, the resulting color of the dyeout obtained with such composition is a high intensity and highly stable red.

As in the production of other oxidative dye compositions, other couplers or primary intermediates may be added to alter the improved color. These compounds, as the skilled artisan will recognize, may be selected from a wide variety of couplers and primary intermediates well known to those skilled in the art. As couplers, there may be mentioned by way of example, 2-hydroxy-4-aminotoluene, 1,3-phenylenediamine, 1,3-diamino-2,6-dimethoxybenzene, 1,3-diamino-6-methoxybenzene, 2,3-dimethoxyphenol, 2,7-dihydroxynaphthalene and 1,3-dihydroxybenzene. One or more of these or other known couplers may be added to the basic compositions.

Typical of the known primary intermediates, at least one of which may be employed in the practice of this invention, are: 1,4-diaminobenzene, N-methyl-1,4-diaminobenzene, 1,4-diamino-2,6-diamethylbenzene, 1,4-diamino-2-hydroxyethylbenzene and N-(2-hydroxypropyl)-1,4-diaminobenzene.

The salts of the foregoing compounds, especially the acid salts such as the monohydrochlorides, the dihydrochlorides or the corresponding sulfuric acid salts may also be employed.

Other conventional agents often employed' in hair coloring compositions may be employed in the dye component or in the developer component. These include, for example, fragrances, coloring agents and chelating agents. Antioxidants such as sodium sulfite, erythorbic acid and ascorbic acid may also be included to inhibit premature oxidation.

Although the dye composition of the invention has been described heretofore as containing the developer so that the oxidative hair dye is produced, the developer can, as is usually the case, be a separate composition which is mixed with the dye precursors, just prior to use. When this is done, the amount of water in the developer composition and in the composition containing the dye precursors and the HLB index of the final composition should be such that the final composition produced by mixing the developer composition and dye precursor composition meets the requirements set forth previously herein for the final dye composition so that the benefits of the invention are obtained.

The developer (oxidant) may be hydrogen peroxide.

The dye and developer components of the compositions of this invention may be prepared in any of the usual formats, such as liquids, lotions, gels and the like. It is of course important that the compositions applied to the hair have and maintain sufficient viscosity to stay on the hair during the coloring procedure.

It is desirable, but not essential, that the viscosities of the dye and developer components be close to each other. If the difference in viscosities is too great they will be difficult to mix. On shaking, the thinner component will aggregate and the rate of blending will be retarded. Typically, the viscosities of the components and of the final compositions will be from about 1000 to 25,000 cps.

The essence of the present invention is the discovery of a limited class of coupler/primary intermediate combinations which in aqueous oxidative dye compositions having the essential characteristics described herein can be employed to dye hair and impart highly desirable colors which manifest unexpectedly improved wash fastness and resistance to light fading compared to hair coloration achievable with conventional oxidative hair coloring compositions and/or permit more intense vivid dyeouts to be obtained.

The total amount of coupler in the oxidative dye compositions of this invention is from about 0.01% to 10%, preferably 0.05% to 5%. This is the same amount of coupler normally employed with conventional oxidative dye compositions. If an auxiliary coupler is employed, from about 0.01 % to 3%, preferably 0.01% to 1.5%, will be a coupler represented by formula I.

The amount of primary intermediate in the final compositions will be the amount normally employed, i.e., from about 0.01% to 10%, preferably 0.1% to 5%. If an auxiliary primary intermediate is employed from about 0.01% to 3%, preferably 0.01% to 1.5%, will be a primary intermediate represented by formula II.

A wide variety of surfactants and surfactant mixtures may be employed in the practice of this invention. Several typically useful surfactants are set forth below. They may be used singly or in blends containing at least two of them.

Useful Amphoteric surfactants include:
1. BETAINES: 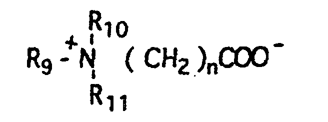
2. SULTAINES: 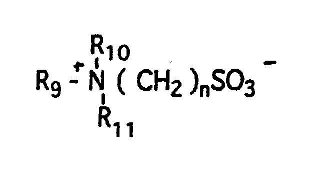
3. PROPIONATES: 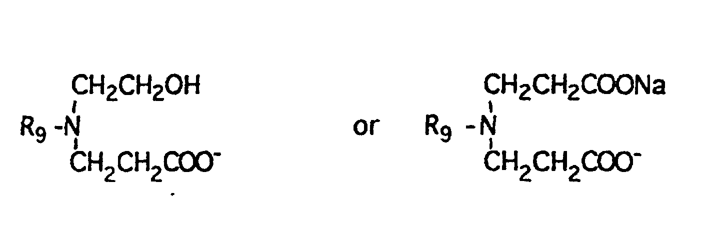
4. GLYCINATES: 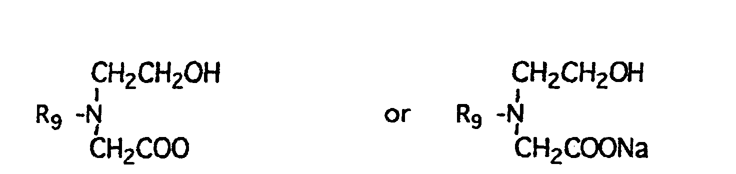
wherein R₉ denotes a C₁-C₆ alkyl or alkylamide group, R₁₀ and R₁₁ which may be the same or different, denote a C₁-C₆ alkyl group or a C₁-C₆ alkyl group substituted with at least one hydroxy group and n is a positive integer from 1 to 5.

Alkylpolyglycosides represented by the formula: 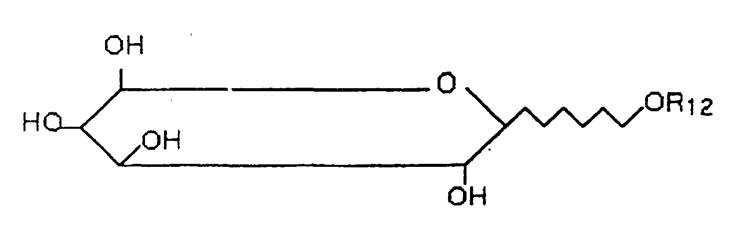 in which R₁₂ denotes a C₁-C₆ alkyl group, are also useful.

Fatty acio soaps such as alkanolamine, alkali metal or alkaline earth metal salt or a carboxylic acid containing from about 11 to 19 carbon atoms, may also be utilized.

Other useful representative surface-active agents include, higher alkylbenzene sulfonates; alkyl-naphthalenesulfonates; sulfonated esters of alcohols and polybasic acids; taurates; fatty alcohol sulfates; sulfates of branched chain or secondary alcohols; alkyldimethylbenzylammonium chlorides, salts of fatty acids or fatty acid mixtures. Illustrative of specific surfactants that can be used are sodium lauryl sulfate; polyoxyethylene lauryl ether: myristyl sulfate; glyceryl monostearate; triethanolamine oleate, sodium salt of palmitic methyl taurine; cetyl pyridinium chloride; lauryl sulfonate; myristyl sulfonate; lauric diethanolamide; polyoxyethylene stearate; ethoxylated oleoyl diethanolamide; stearyldimethyl benzyl ammonium chloride; dodecylbenzene sodium sulfonate; triethanolamine salt of p-dodecylbenzene sulfonate; nonylnaphthalene sodium sulfonate; dioctyl sodium sulfosuccinate; sodium N-methyl-N-oleoyl taurate; oleic acid ester of sodium isothionate; sodium dodecyl sulfate; the sodium salt of 3-diethyl tridecanol-6-sulfate and the like.

If a thickening agent is employed to control the viscosity of the composition, two classes of these products have been found to be useful. One is a modified polyurethane of which several are available. They are sold under the trade names Aculyn 44 and Aculyn 46 (Rohm & Haas). The other is of the class of hydrophobicly modified polyacrylic acids sold as Steareth-10 alkyl ether/acrylate polymer (Aculyn 22 from Rohm & Haas), acrylate copolymer (Aculyn 33 from Rohm & Haas), and ceteareth-20 acrylates/steareth-20 methacrylate copolymer. The latter class is employed with an alkaline reagent.

Any of a wide variety of alkaline reagents may be employed with the modified polyacrylic acids. Because of its freedom from toxicity over a wide concentration range and its economy, ammonium hydroxide is an acceptable alkalizing agent. However, any other compatible ammonia derivative can be used in place of, or together with ammonium hydroxide to effect the desired alkalinity. For example, an alkylamine such as ethylamine, or triethylamine; or alkanolamines, such as ethanolamine, diethanolamine, aminomethyl propanol, aminomethyl propanediol and trishydroxymethyl aminomethane may be employed. Likewise, any other of the organic or inorganic alkalizing agents may be used, such as sodium or potassium hydroxide, sodium or potassium carbonate, sodium phosphate, sodium hydrogen phosphate, sodium silicate, guanidine hydroxide and the like. The preferred alkaline reagents are ammonium hydroxide, sodium carbonate and ethanolamine.

When the alkaline reagents listed above, or their equivalents, are employed at a concentration of from about 0.5% to 10%, the pH of the dye component will be from about 8 to 10.5.

Although they are not essential, buffering agents may be employed to stabilize the pH of the dye component during storage. Typically useful buffers include ammonium and alkali metal phosphates, bicarbonates, carbonates citric acid, fatty acids and, to a lesser extent, borates. Also suitable are amino buffers such as, N-[2-hydroxyethyl]-piperazine-N¹-[2-ethanesulfonic acid] (HEPES), N-[2-acetamido]-2-aminoethane sulfonic acid (ACES), tris[-hydroxymethyl] aminomethane (TRIS) and N-tris[hydroxymethyl]-methyl-3-aminopropane sulfonic acid (TAPS). The ammonium and alkali metal carbonates are also suitable. The preferred buffers are TRIS, sodium and potassium carbonate, bicarbonate and phosphate.

The dye component and the developer can be combined by mixing them directly on the hair of the user. If they are mixed on the hair, adding the dye component first is preferable. It is preferred to mix them in a mixing vessel for subsequent application to the hair.

The kit provided according to this invention comprises at least two containers, one containing the dye component, the other the developer component.

The method of the invention comprises applying the oxidative dye composition to the hair to be colored and allowing it to remain in contact with the hair until the desired hair color has been attained after which the composition is removed from the hair by rinsing.

Some typical examples of dye components and developer components are shown in Tables 1 and 2. Each of the components was mixed using conventional procedures. Table 3 records the color results on gray hair when a dye component of Table 1 was mixed with a developer of Table 2 in a 1 to 1 ratio and the resultant dye composition was applied to human hair for 30 minutes employing the usual procedures for human hair dyeing.

Referring once again to Figure 1, in generating the data for such Figure oxidative dye compositions were used to dye hair. The dye precursors and oxidant employed in producing the dye compositions were fixed. In each case the final dye composition employed contained:
0.25 % 1-acetoxy-2-methyl naphthalene,
0.2 % 3-methyl-p-aminophenol and
3% Hydrogen peroxide.

The Cumulative HLB Indexes of the final dye compositions were varied by varying the amount and/or nature of the non-dye producing composition ingredients (i.e. the solvent(s) and/or surfactant(s)).

The Cumulative HLB Index was calculated for each dye composition and red intensity (CIE "a" value) was measured for each composition's dyeout.

As noted earlier, in Figure 1 the Cumulative HLB Indexes were plotted against CIE "a" value.

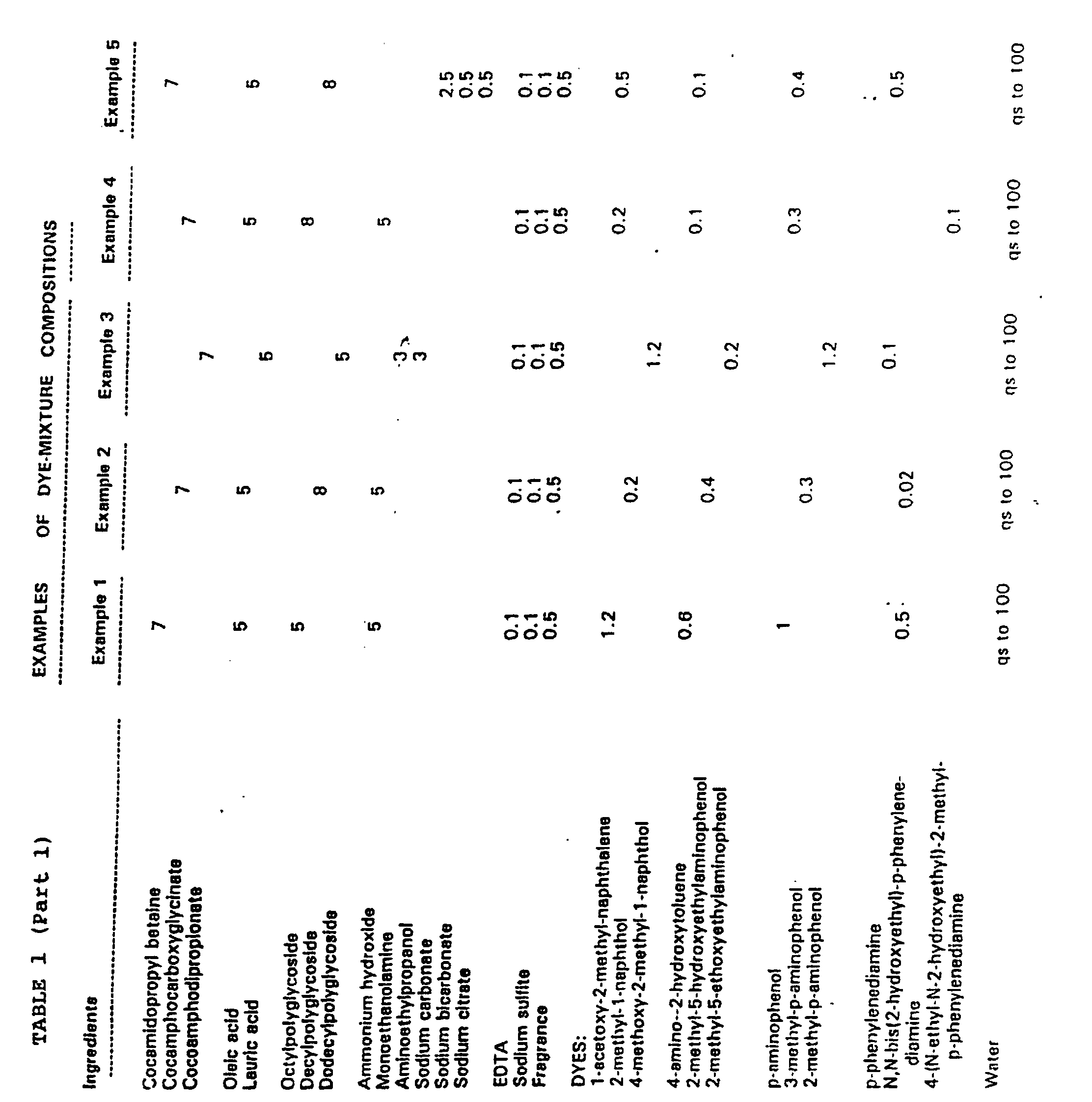

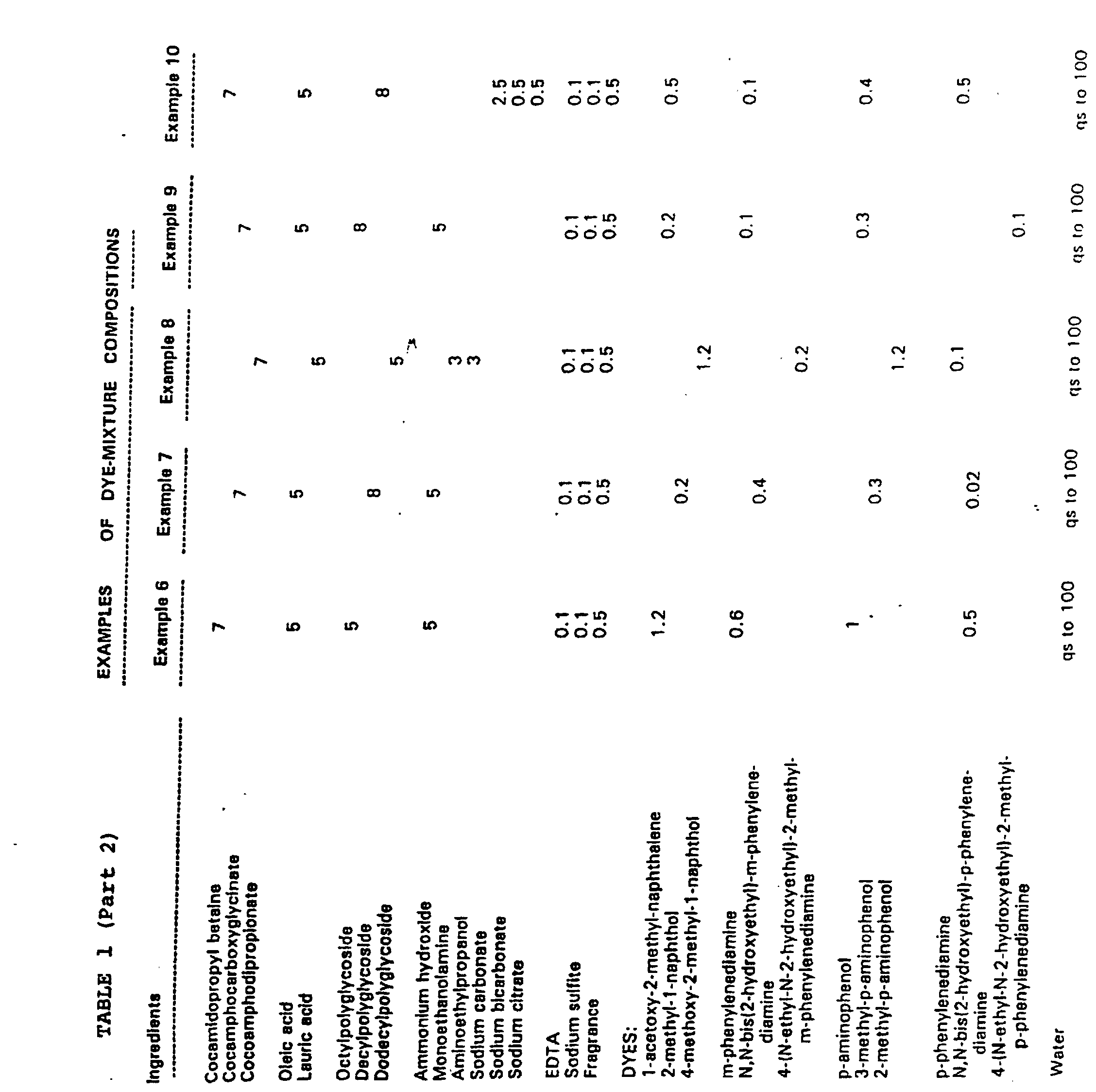

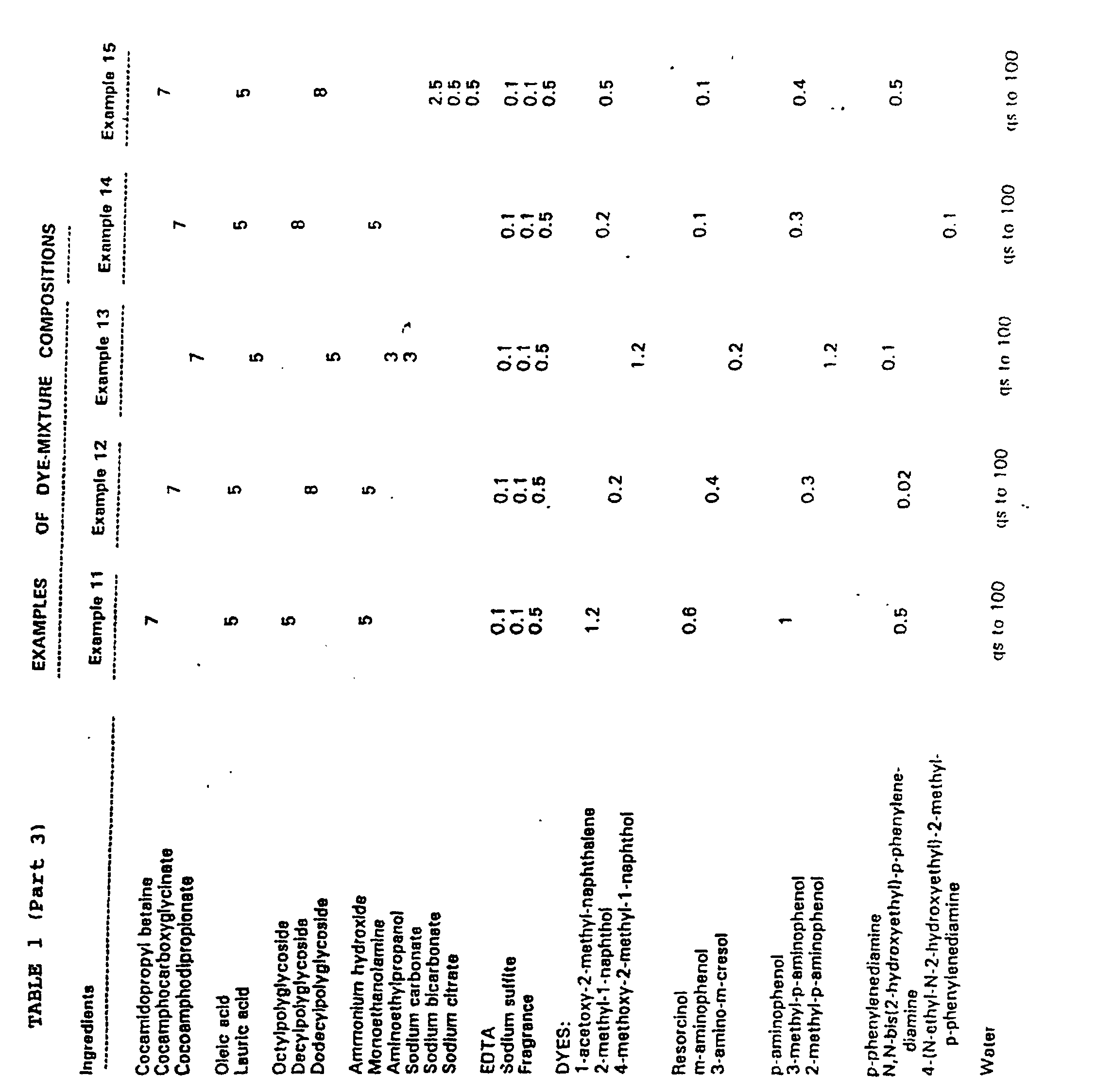

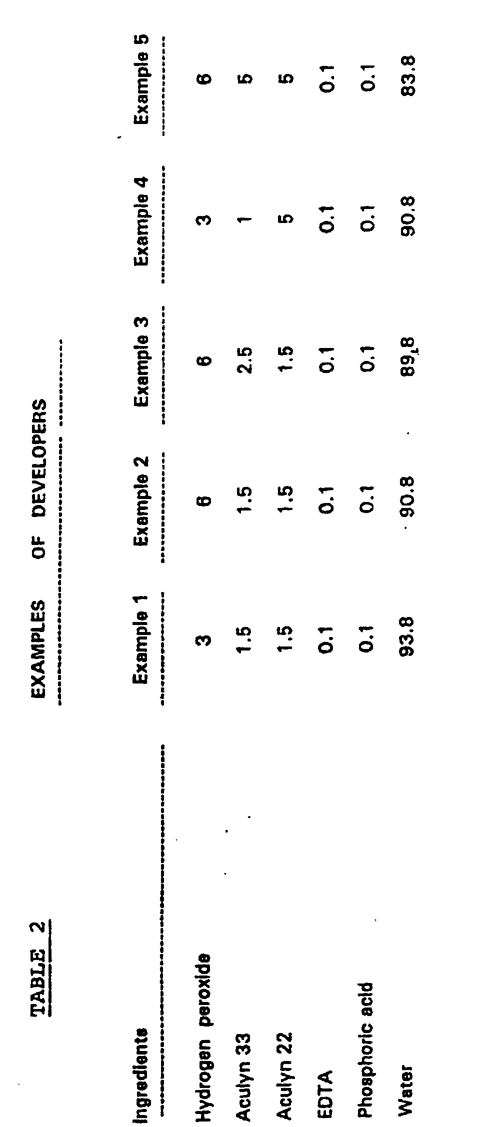

**TABLE 3**

| DYE-MIXTURE | DEVELOPER | DYEOUT COLOR ON GRAY HAIR |
|---|---|---|
| 1 | 2 | Dark burgundy |
| 2 | 3 | Light burgundy |
| 3 | 3 | Bright red |
| 4 | 4 | Copper red |
| 5 | 1 | Reddish brown |
| 6 | 3 | Dark burgundy |
| 7 | 5 | Burgundy |
| 8 | 2 | Dark auburn |
| 9 | 1 | Auburn brown |
| 10 | 1 | Burgundy brown |
| 11 | 5 | Dark auburn |
| 12 | 5 | Light copper brown |
| 13 | 4 | Auburn |
| 14 | 3 | Copper brown |
| 15 | 4 | Warm ash brown |

All of the above dyeouts were made by mixing one part of the dye composition with one part of a developer composition and treating tresses of gray hair with the mixture for 20 minutes.

## Claims

1. A kit for oxidative hair dyeing comprising a first container containing a coupler, at least one primary intermediate, water and at least one surfactant, and a second container containing an oxidant, wherein the coupler is a compound of the formula I: 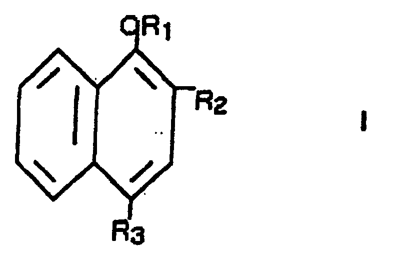 wherein R₁ denotes hydrogen or a C₁-C₆ acyl group, R₂ denotes a C₁-C₈ alkyl group or a C₁-C₈ alkyl group substituted with at least one hydroxyl group, and R₃ denotes hydrogen, a C₁-C₆ alkyl group or a C₁-C₆ alkyl group substituted with at least one hydroxyl group; and the primary intermediate is a compound of the formula II: 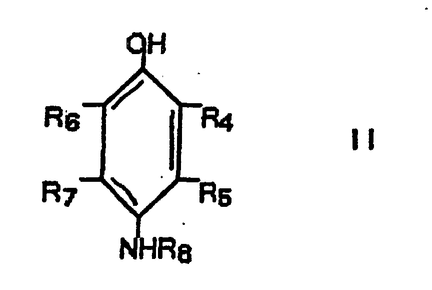 wherein R₄, R₅, R₆ and R₇, independently of one another, denote hydrogen, a C₁-C₆ alkyl group or a C₁-C₆ alkyl group substituted with at least one hydroxyl group and R₈ denotes hydrogen or a C₁-C₆ alkyl group; and the Cumulative HLB Index of the composition formed by mixing the contents of the first and second containers is 2.5 or less.

2. The kit of Claim 1, wherein said Cumulative HLB Index is from 2.1 to 2.5.

3. The kit of Claim 1, wherein the oxidant is hydrogen peroxide.

4. The kit of Claim 1, wherein said composition contains from 0.01 % to 10% by weight of the coupler of formula I and from 0.01% to 10% by weight of the primary intermediate of formula II.

5. A method of oxidative hair dyeing comprising reacting a 2-substituted-1-naphthol of formula I: 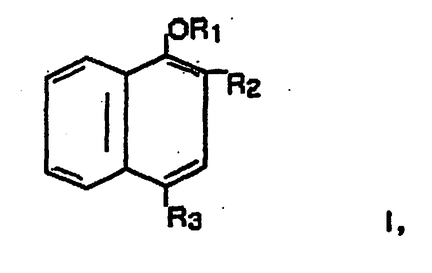 wherein R₁ denotes hydrogen or a C₁-C₈ acyl group, R₂ denotes a C₁-C₈ alkyl group or a C₁-C₈ alkyl group substituted with at least one hydroxyl group, and R₃ denotes hydrogen, a C₁-C₆ alkyl group or a C₁-C₈ alkyl group substituted with at least one hydroxyl group, with a primary intermediate of formula II: 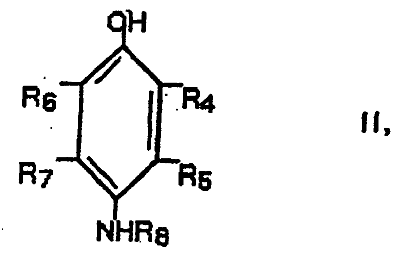 wherein R₄, R₅, R₆ and R₇, independently of one another, denote hydrogen, a C₁-C₆ alkyl group or a C₁-C₆ alkyl group substituted with at least one hydroxyl group and R₈ denotes hydrogen or a C₁-C₆ alkyl group, in a composition comprising an oxidant and at least one surfactant, wherein the Cumulative HLB Index of the composition is 2.5 or less.

6. The method of Claim 5, wherein the Cumulative HLB Index is from 2.1 to 2.5.

7. The method of Claim 5, wherein the oxidant is hydrogen peroxide.

8. The method of Claim 5, wherein the composition contains from 0.01% to 10% by weight of the coupler of formula I and from 0.01% to 10% of the primary intermediate of formula II, based on the total weight of the composition.

## Patentansprüche

1. Ausstattung bzw. Kit für die oxidative Haarfärbung, umfassend einen ersten Behälter, enthaltend ein Kupplungsmittel, mindestens ein primäres Intermediat, Wasser und mindestens ein oberflächenaktives Mittel, und zweiten Behälter, enthaltend ein Oxidationsmittel, worin das Kupplungsmittel eine Verbindung der Formel I ist: 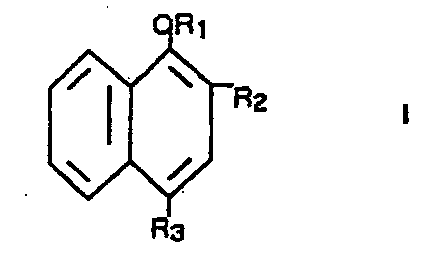 worin R₁ Wasserstoff oder eine C₁-C₆-Acylgruppe bezeichnet, R₂ eine C₁-C₆-Alkylgruppe oder eine mit mindestens einer Hydroxylgruppe substituierte C₁-C₆-Alkylgruppe bezeichnet und R₃ Wasserstoff, eine C₁-C₆-Alkylgruppe oder eine mit mindestens einer Hydroxylgruppe substituierte C₁-C₆-Alkylgruppe bezeichnet; und worin das primäre Intermediat eine Verbindung der Formel II ist: 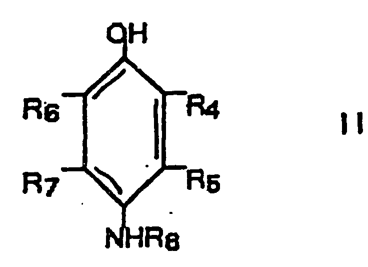 worin R₄, R₅, R₆ und R₇ unabhängig voneinander bezeichnen Wasserstoff, eine C₁-C₆-Alkylgruppe oder eine mit mindestens einer Hydroxylgruppe substituierte C₁-C₆-Alkylgruppe und worin R₈ bezeichnet Wasserstoff oder eine C₁-C₆-Alkylgruppe, und worin der Gesamt-HLB-Index der Zusammensetzung, die durch Mischen der Inhalte des ersten und des zweiten Behälters gebildet wird, 2,5 oder kleiner ist.

2. Ausstattung gemäß Anspruch 1, worin der Gesamt-HLB-Index von 2,1 bis 2,5 beträgt.

3. Ausstattung gemäß Anspruch 1, worin das Oxidationsmittel Wasserstoffperoxid ist.

4. Ausstattung gemäß Anspruch 1, worin die Zusammensetzung von 0,01 % bis 10 Gew.-% des Kupplungsmittels der Formel I und von 0,01 % bis 10 Gew.-% des primären Intermediats der Formel II enthält.

5. Verfahren zur oxidativen Haarfärbung, umfassend das Umsetzen eines 2-substituierten 1-Naphthols der Formel I: 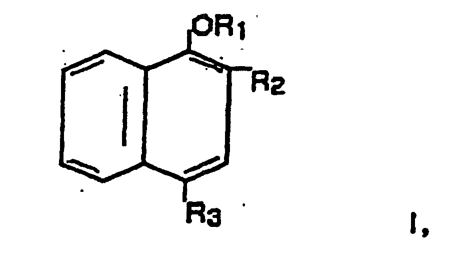 worin R₁ Wasserstoff oder eine C₁-C₆-Acylgruppe bezeichnet, R₂ eine C₁-C₆-Alkylgruppe oder eine mit mindestens einer Hydroxylgruppe substituierte C₁-C₆-Alkylgruppe bezeichnet und R₃ Wasserstoff, eine C₁-C₆-Alkylgruppe oder eine mit mindestens einer Hydroxylgruppe substituierte C₁-C₆-Alkylgruppe bezeichnet, mit einem primären Intermediat der Formel II: 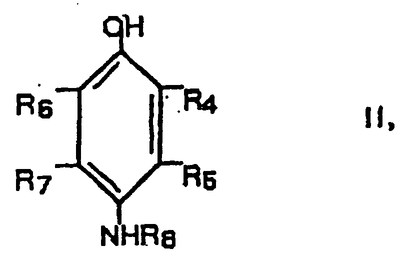 worin R₄, R₅, R₆ und R₇ unabhängig voneinander bezeichnen Wasserstoff, eine C₁-C₆-Alkylgruppe oder eine mindestens einer Hydroxylgruppe substituierte C₁-C₆-Alkylgruppe und worin R₈ Wasserstoff oder eine C₁-C₆-Alkylgruppe bezeichnet, in einer Zusammensetzung, umfassend ein Oxidationsmittel und mindestens ein oberflächenaktives Mittel, worin der Gesamt-HLB-Index der Zusammensetzung 2,5 oder kleiner ist.

6. Verfahren gemäß Anspruch 5, worin der Gesamt-HLB-Index von 2,1 bis 2,5 beträgt.

7. Verfahren gemäß Anspruch 5, worin das Oxidationsmittel Wasserstoffperoxid ist.

8. Verfahren gemäß Anspruch 5, worin die Zusammensetzung von 0,01 % bis 10 Gew.-% des Kupplungsmittels der Formel I und von 0,01 % bis 10 % des primären Intermediats der Formel II enthält, basierend auf dem Gesamtgewicht der Zusammensetzung.

## Revendications

1. Kit de coloration pour cheveux par oxydation comprenant un premier récipient contenant un agent de couplage, au moins un intermédiaire primaire, de l'eau et au moins un agent tensioactif, et un second récipient contenant un oxydant, dans lequel l'agent de couplage est un composé de formule I : 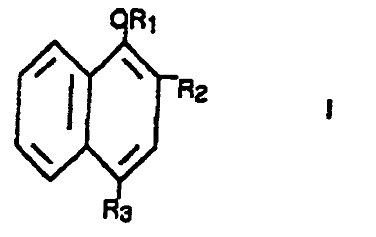 dans laquelle R₁ représente hydrogène ou un groupe acyle en C₁-C₆, R₂ représente un groupe alkyle en C₁-C₆ ou un groupe alkyle en C₁-C₆ substitué par au moins un groupe hydroxyle, et R₃ représente hydrogène, un groupe alkyle en C₁-C₆ ou un groupe alkyle en C₁-C₆ substitué par au moins un groupe hydroxyle ; et l'intermédiaire primaire est un composé de formule II : 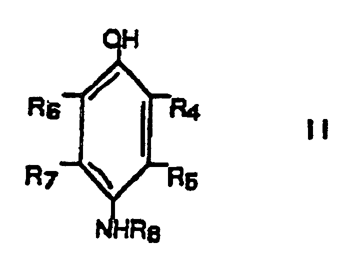 dans laquelle R₄, R₅, R₆ et R₇, indépendamment l'un de l'autre, représentent hydrogène, un groupe alkyle en C₁-C₆ ou un groupe alkyle en C₁-C₆ substitué par au moins un groupe hydroxyle et R₈ représente hydrogène ou un groupe alkyle en C₁-C₆ ; et l'indice HLB cumulé de la composition formée en mélangeant le contenu du premier et du second récipients est de 2,5 ou moins.

2. Kit selon la revendication 1, dans lequel ledit indice HLB cumulé est de 2,1 à 2,5.

3. Kit selon la revendication 1, dans lequel l'oxydant est du peroxyde d'hydrogène.

4. Kit selon la revendication 1, dans lequel ladite composition contient de 0,01 à 10 % en poids de l'agent de couplage de formule I et de 0,01 à 10 % en poids de l'intermédiaire primaire de formule II.

5. Procédé de coloration de cheveux par oxydation consistant à faire réagir un 1-naphtol substitué m2 de formule I : 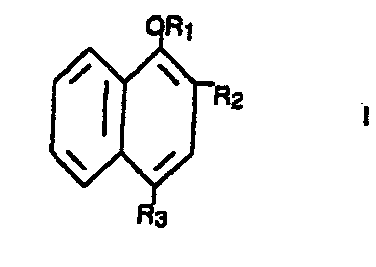 dans laquelle R₁ représente hydrogène ou un groupe acyle en C₁-C₆, R₂ représente un groupe alkyle en C₁-C₆ ou un groupe alkyle en C₁-C₆ substitué par au moins un groupe hydroxyle, et R₃ représente hydrogène, un groupe alkyle en C₁-C₆ ou un groupe alkyle en C₁-C₆ substitué par au moins un groupe hydroxyle, avec un intermédiaire primaire de formule II : 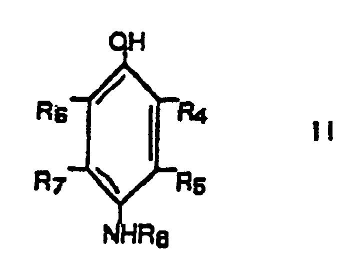 dans laquelle R₄, R₅, R₆ et R₇, indépendamment l'un de l'autre, représentent hydrogène, un groupe alkyle en C₁-C₆ ou un groupe alkyle en C₁-C₆ substitué par au moins un groupe hydroxyle et R₈ représente hydrogène ou un groupe alkyle en C₁-C₆, dans une composition comprenant un oxydant et au moins un agent tensioactif, l'indice HLB cumulé de la composition étant de 2,5 ou moins.

6. Procédé selon la revendication 5, dans lequel ledit indice HLB cumulé est de 2,1 à 2,5.

7. Procédé selon la revendication 5, dans lequel l'oxydant est du peroxyde d'hydrogène.

8. Procédé selon la revendication 5, dans lequel la composition contient de 0,01 à 10 % en poids de l'agent de couplage de formule I et de 0,01 à 10 % de l'intermédiaire primaire de formule II, sur la base du poids total de la composition.
